(19) Europäisches Patentamt
European Patent Office
Office européen des brevets

(11) Numéro de publication : **0 504 017 A1**

# (12) DEMANDE DE BREVET EUROPEEN

(21) Numéro de dépôt : **92400577.0**

(22) Date de dépôt : **06.03.92**

(51) Int. Cl.[5] : **C07D 405/12,** A61K 31/35

(30) Priorité : **08.03.91 FR 9102799**

(43) Date de publication de la demande :
**16.09.92 Bulletin 92/38**

(84) Etats contractants désignés :
**AT BE CH DE DK ES FR GB GR IT LI LU NL PT SE**

(71) Demandeur : **ADIR ET COMPAGNIE**
**1 rue Carle Hébert**
**F-92415 Courbevoie Cédex (FR)**

(72) Inventeur : **Le Baut, Guillaume**
**5 rue de la Baugerie**
**F-44230 Saint Sebastien sur Loire (FR)**
Inventeur : **Babingui, Jean-Paul**
**10, rue de Briord**
**F-44000 Nantes (FR)**
Inventeur : **Robert, Jean-Michel**
**145 rue Bonne Garde**
**F-44000 Nantes (FR)**
Inventeur : **Renard, Pierre**
**50 avenue de Villeneuve l'Etang**
**F-78000 Versailles (FR)**
Inventeur : **Renaud de la Faverie, Jean-François**
**7, rue des Erables - Rocquencourt**
**F-78150 Le Chesnay (FR)**
Inventeur : **Adam, Gérard**
**9 Clos du Mesnil, Route du Pecq**
**F-78600 Le Mesnil le Roi (FR)**

(54) **Dérivés du benzopyrano, leur procédé de préparation et les compositions pharmaceutiques qui les contiennent.**

(57) Procédé de préparation des composés de formule I :

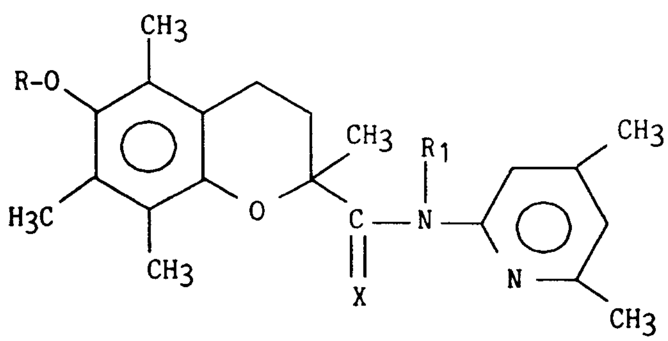

I

dans laquelle :

$R_1$ représente un atome d'hydrogène ou un radical alkyle comprenant de 1 à 3 atomes de carbone,

R représente un atome d'hydrogène, un radical alkyle comprenant de 1 à 4 atomes de carbone, ou un radical acyle - CO - R′ dans lequel R′ représente un radical alkyle comportant de 1 à 4 atomes de carbone,

X représente O ou $H_2$,

de leurs isomères,

ainsi que de leurs sels d'addition à un acide ou à une base pharmaceutiquement acceptable.

EP 0 504 017 A1

La présente invention concerne de nouveaux dérivés du benzopyrane, leur procédé de préparation et les compositions pharmaceutiques qui les contiennent

On connait de nombreux dérivés du benzopyrane, notamment les tocophérols, composés vitaminiques E dotés en particulier de propriétés antioxydantes. De nombreux dérivés du tocophérol ont été préparés, résultant d'une modification de la chaine latérale de nature alcanoïque, sans augmentation de l'activité antioxydante. D'autres dérivés benzopyraniques, comme l'acide (6 - hydroxy 2, 5, 7, 8 - tétraméthylchroman - 2 - yl) carboxylique (J. Am. Oil Chem. Soc. 1974, 51, 200) possèdent une bonne activité antioxydante, mais uniquement à visée industrielle, n'ayant pas reçu d'applications thérapeutiques.

Plus récemment, la demande de brevet WO 88/08424 a décrit d'autres dérivés de l'acide chroman - 2 - yl carboxylique et plus généralement d'acides (chroman - 2 - yl)alkylcarboxyliques, présentant des propriétés anti-oxydantes intéressantes.

La demanderesse a maintenant découvert de nouveaux dérivés du benzopyrane, et plus particulièrement de l'acide (benzopyran - 2 - yl) carboxylique ou acide (chroman - 2 - yl)carboxylique possédant une activité anti-oxydante nettement supérieure à celles de la demande WO 88/08424 qui constitue l'art antérieur le plus proche.

Plus spécifiquement, l'invention concerne de nouveaux dérivés du benzopyrane répondant à la formule générale I

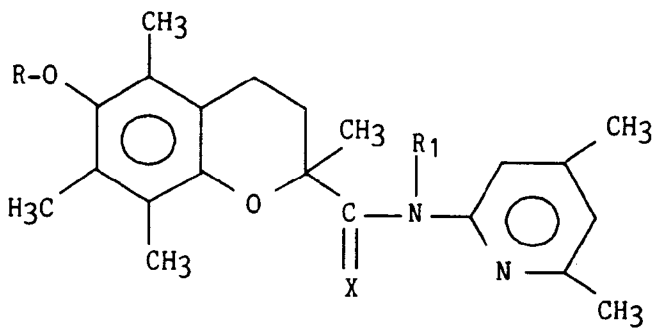

I

dans laquelle :

$R_1$ représente un atome d'hydrogène ou un radical alkyle comprenant de 1 à 3 atomes de carbone,

R représente un atome d'hydrogène, un radical alkyle comprenant de 1 à 4 atomes de carbone, ou un radical acyle - CO - R′ dans lequel R′ représente un radical alkyle comportant de 1 à 4 atomes de carbone,

X représente O ou $H_2$,

leurs isomères,

ainsi que, lorsque X représente $H_2$, leurs sels d'addition à un acide pharmaceutiquement acceptable, et lorsque R représente un atome d'hydrogène, leurs sels d'addition à une base pharmaceutiquement acceptable.

Parmi les acides ou les bases pharmaceutiquement acceptables que l'on peut utiliser pour salifier les composés de l'invention, on peut citer, à titre non limitatif, les acides chlorhydrique, bromhydrique, sulfurique, nitrique, oxalique, malique, maléique, succinique, tartrique, méthanesulfonique, camphorique, camphosulfonique, la soude, la potasse, la triéthylamine, la diéthylamine, l'éthanolamine, l'arginine, la lysine, la diéthanolamine...

L'invention s'étend également au procédé d'obtention des dérivés de formule I, caractérisé en ce que l'on estérifie l'acide (6 - hydroxy - 2, 5, 7, 8 - tétraméthylchroman - 2 - yl) carboxylique, en milieu basique anhydre par un anhydride d'acide de formule R′ - CO - O - CO - R′, ou un halogénure d'acide de formule R′ - CO - Hal, formules dans lesquelles R′ a la même signification que dans la formule I et Hal représente un atome d'halogène, pour obtenir un acide de formule II

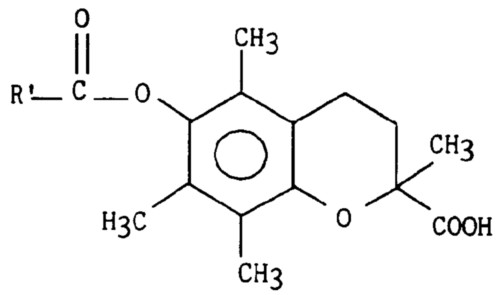

II

dans laquelle R′ a la même définition que dans la formule I, qui est transformé en son chlorure par action du chlorure de thionyle en milieu anhydre, puis traité, dans un solvant approprié en présence d'un agent alcalin, par une amine de formule III

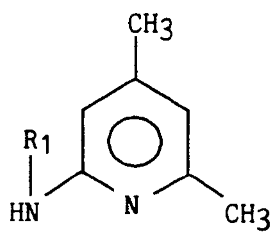

III

dans laquelle $R_1$ a la même signification que dans la formule I, pour obtenir un composé de formule $I_a$

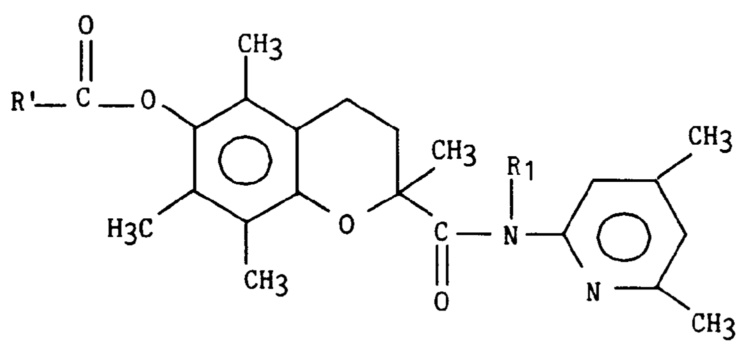

$I_a$

dans laquelle R′ et $R_1$ sont tels que définis précédemment, cas particulier des composés de formule I pour lesquels R représente - CO - R′ et X représente un atome d'oxygène,

qui est ensuite saponifié par action d'un hydroxyde d'un métal alcalin ou alcalino-terreux en un composé de formule $I_b$

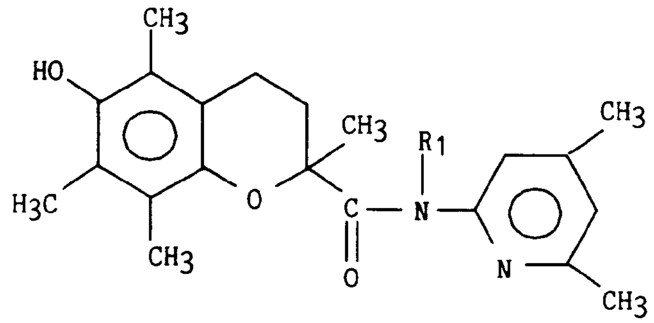

$I_b$

dans laquelle $R_1$ est tel que défini précédemment, cas particulier des composés de formule I pour lesquels R représente un atome d'hydrogène et X un atome d'oxygène qui peut être ensuite :

– soit transformé en son sel d'addition à une base pharmaceutiquement acceptable,

– soit éthérifié par un dérivé halogéné de formule $R_2$ - Hal, dans laquelle $R_2$ représente un radical alkyle de 1 à 4 atomes de carbone pour obtenir un composé de formule $I_c$

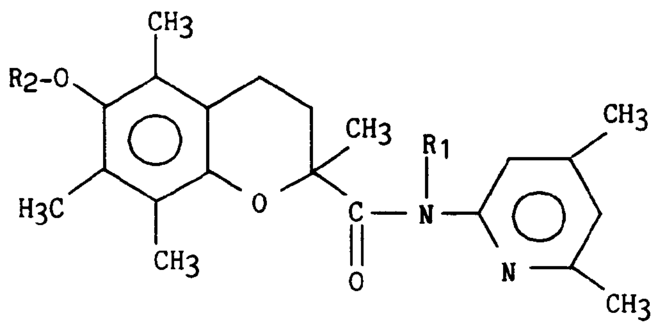

$I_c$

dans laquelle $R_1$ et $R_2$ sont tels que définis précédemment, cas particulier des composés de formule I pour lesquels X représente un atome d'oxygène et R représente un groupement $R_2$ tel que défini précédemment,

– soit réduit par action d'un hydrure mixte de métal alcalin en un dérivé de formule $I_d$

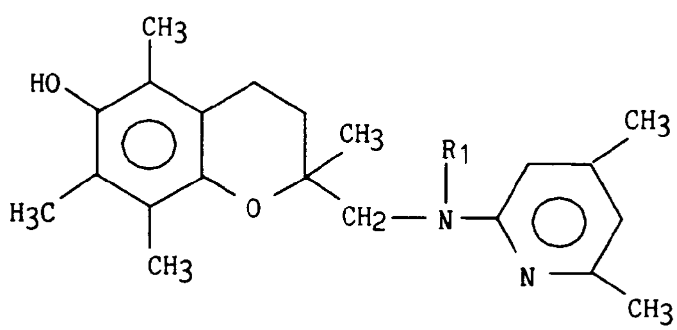

$I_d$

dans laquelle $R_1$ est tel que défini précédemment, cas particulier des composés de formule I pour lesquels R représente un atome d'hydrogène et X représente $H_2$,

que l'on peut,

– ou bien salifier par action d'une base ou d'un acide pharmaceutiquement acceptable,

– ou bien éthérifier ou estérifier par action d'un dérivé de formule $R'_2$-Hal dans laquelle Hal représente un atome d'halogène et $R'_2$ représente un radical alkyle de 1 à 4 atomes de carbone ou un radical acyle - CO - R' tel que défini dans la formule I, en un dérivé de formule $I_e$

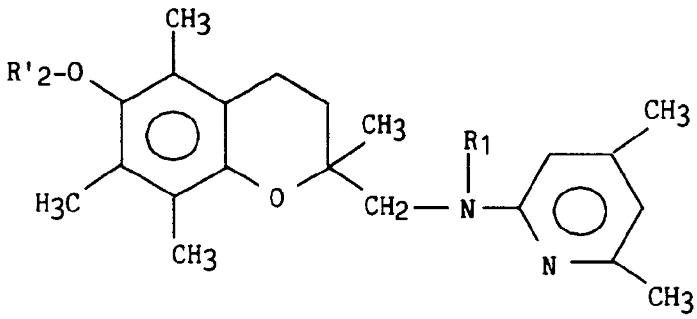

Ie

dans laquelle R et $R'_2$ sont tels que définis précédemment, cas particulier des composés de formule I pour lesquels X représente $H_2$ et R possède les significations de $R'_2$, qui est, si l'on désire, salifié par action d'un acide pharmaceutiquement acceptable.

Les composés de formule $I_a$, $I_b$, $I_c$, $I_d$, et $I_e$ forment l'ensemble des composés de formule I, qui peuvent être, si l'on désire, séparés en leurs isomères selon des techniques classiques.

Comparés aux composés de l'art antérieur, les composés de la présente invention possèdent de façon surprenante des propriétés antioxydantes très importantes. Les études pharmacologiques ont notamment montré que ces composés étaient doués d'activités protectrices remarquables dans le cadre des processus de peroxydations des lipides cellulaires et des lipoprotéines de faible densité (LDL).

Ces activités sont 100 fois supérieures à celle du composé le plus proche de l'art antérieur, c'est à dire l'exemple 102 de la demande WO 88/08424 de formule (a) :

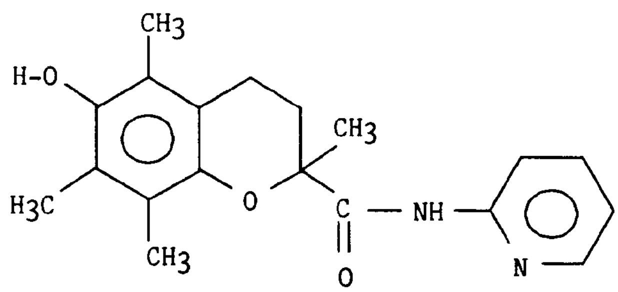

(a)

Par ailleurs, les composés de la présente invention présentent la particularité d'avoir un puissant effet inhibiteur sur la biosynthèse des prostanoïdes, effet que ne possède pas le composé le plus proche de l'art antérieur.

On peut donc attendre des composés de l'invention, qui présentent à la fois des propriétés inhibitrices de la peroxydation lipidique et de la biosynthèse des prostanoïdes, une action particulièrement nouvelle et bénéfique dans les affections où interviennent non seulement une peroxydation des lipides membranaires mais aussi un dérèglement de la synthèse des prostanoïdes.

Les composés de la présente invention peuvent ainsi être utilisés dans le traitement ou la prévention des affections dues ou reliées à de tels phénomènes de peroxydation et de dérèglements et notamment les désordres ischémiques centraux ou périphériques, les maladies inflammatoires comme l'arthrite rhumatoïde, les maladies métaboliques comme l'athérome et l'artériosclérose, les maladies respiratoires comme l'asthme ou l'emphysème, les maladies d'origine immunologique comme le lupus érythémateux, les réactions allergiques, certains cancers, le vieillissement cérébral ou cutané ainsi que dans la prévention et le traitement des dommages dus aux traumatismes chirurgicaux tels que la reperfusion d'organes.

La présente invention a également pour objet les compositions pharmaceutiques contenant les dérivés de formule I, ou un de leurs sels d'addition à un acide ou une base pharmaceutiquement acceptable, seul ou en combinaison avec un ou plusieurs excipients inertes non toxiques.

Parmi les compositions pharmaceutiques selon l'invention, on pourra citer plus particulièrement celles qui conviennent à l'administration orale, parentérale, nasale, rectale, perlinguale, oculaire, ou pulmonaire et notamment les préparations injectables, les aérosols, les gouttes oculaires ou nasales, les comprimés simples, pelliculés ou dragéifiés, les gélules, les capsules, les crèmes, pommades, gels dermiques,...

La posologie utile varie selon l'âge et le poids du patient, la voie d'administration, la nature de l'affection et des traitements éventuellement associés et s'échelonne entre 0,5 mg et 2 grammes par 24 heures.

Les exemples qui suivent illustrent l'invention et ne la limitent en aucune façon.

La matière première est décrite dans la littérature.

**Exemple 1 : N - (4,6 - diméthylpyridin - 2 - yl) (6 - acétoxy - 2, 5, 7, 8 - tétraméthylchroman - 2 - yl) carboxamide**

**STADE A : Acide (6 - acétoxy 2, 5, 7, 8 - tétraméthylchroman - 2 - yl) carboxylique**

Dissoudre 50 grammes (0,2 mole) d'acide (6 - hydroxy - 2, 5, 7, 8 tétraméthylchroman - 2 - yl) carboxylique dans 150 $cm^3$ de pyridine anhydre. Ajouter goutte à goutte 9,4 $cm^3$ (0,1 mole) d'anhydride acétique sous courant d'azote. Agiter pendant 2 heures à une température de 30°C. Après refroidissement, verser le mélange sur de la glace, extraire le produit attendu par de l'éther éthylique, laver la phaqe organique par une solution d'acide chlorhydrique 0,2 N, puis à l'eau jusqu'à neutralité. Après évaporation du solvant, on recueille une masse huileuse qui cristallise après trituration dans l'éther diisopropylique.

**STADE B : N - (4,6 - diméthylpyridin - 2 - yl) (6 - acétoxy - 2, 5, 7, 8 - tétraméthylchroman - 2 - yl) carboxamide**

Disssoudre 5,4 grammes (18,47 mmoles) du composé obtenu au stade A précédent dans 30 $cm^3$ de benzène anhydre, ajouter 2 $cm^3$ (27,41 mmole) de chlorure de thionyle, chauffer à reflux pendant 3 heures, chasser le solvant sous vide en éliminant l'excès de chlorure de thionyle. Dissoudre le chlorure d'acide ainsi obtenu dans 30 $cm^3$ de dichloroéthane

Dans un autre récipient, dissoudre 2,26 grammes (18,5 mmole) de 2 - amino - 4,6 - diméthylpyridine dans 20 $cm^3$ de dichloroéthane, ajouter 7,7 $cm^3$ de triéthylamine et verser goutte à goutte à ce mélange la solution de chlorure d'acide précédente. Après 8 heures d'agitation, évaporer le solvant sous vide, reprendre le résidu par 30 $cm^3$ d'eau, neutraliser par une solution de $NaHCO_3$, extraire par du chlorure de méthylène, laver la phase organique à l'eau, puis la sécher sur sulfate de sodium. Après évaporation du solvant, purifier par chromatographie sur colonne de gel de silice en éluant par du chlorure de méthylène.

On obtient le produit attendu (rendement 71 %)

- Point de fusion : 128 - 129°C
- Caractéristiques spectrales en IR :

  νNH : 3410, 3390 $cm^{-1}$
  νCO ester : 1760 $cm^{-1}$
  νCO amide 1690 $cm^{-1}$

**Exemple 2 : N - (4,6 - diméthylpyridin - 2 - yl) (6 - hydroxy - 2, 5, 7, 8 - tétraméthylchroman - 2 - yl) carboxamide**

Dissoudre 5,25 grammes (13,24 mmole) du composé de l'exemple 1 dans 80 $cm^3$ d'éthanol, ajouter 40 $cm^3$ d'eau, puis 3,18 grammes d'hydroxyde de sodium dissous dans 10 $cm^3$ d'eau et 10 $cm^3$ d'éthanol. Agiter sous azote pendant 3 heures, diluer le mélange par de l'eau acidifiée par de l'acide acétique, filtrer, laver à l'eau, puis à l'éther, sécher et évaporer. On obtient 4,49 grammes du composé de l'exemple (rendement 96%).

- Point de fusion : 244 - 245°C
- Caractéristiques spectrales en IR :

  νNH : 3380 $cm^{-1}$
  νC-H : 2990, 2980, 2930 $cm^{-1}$
  νC = O : 1700 $cm^{-1}$

**Exemple 3 : N - (4,6 - diméthylpyridin - 2 - yl) (6 - méthoxy - 2, 5, 7, 8 - tétrathylchroman - 2 - yl) carboxamide**

A une suspension de 2,2 grammes (6,2 mmole) du composé de l'exemple 2 dans 40 $cm^3$ de diméthylformamide, ajouter à une température de 0°C 0,25 grammes (6,25 mmole) d'hydrure de sodium à 60 %. Agiter pendant une heure puis verser 0,4 $cm^3$ (6,42 mmole) d'iodure de méthyle en solution dans 5 $cm^3$ de diméthylformamide, maintenir l'agitation pendant 90 minutes. Verser la solution sur de la glace, acidifier par de l'acide acétique, extraire par du chlorure de méthylène, laver la phase organique à l'eau, sécher sur sulfate de sodium,

évaporer le solvant et purifier le résidu huileux obtenu par chromatographie sur gel de silice en éluant par du chlorure de méthylène.

On obtient 1,62 grammes du composé de l'exemple (rendement 70,8 %).

– Point de fusion : 97 - 98°C
– Caractéristiques spectrales en IR :
  νNH : 3400, 3360 $cm^{-1}$
  νCH : 2980, 2920 $cm^{-1}$
  νCO : 1680 $cm^{-1}$
  νNH : 1520 $cm^{-1}$

**Exemples 4 à 6 :**

En remplaçant, dans l'exemple 1, stade B, la 2 - amino - 4,6 diméthylpyridine par la N - (4,6 - diméthylpyridin - 2 - yl) éthylamine, puis en opérant comme dans les exemples 2 et 3, on obtient successivement :

**Example 4 : N - éthyl N - (4,6 - diméthylpyridin - 2 - yl) (6 - acétoxy - 2, 5, 7, 8 - tétraméthylchroman - 2 - yl) carboxamide**

Rendement : 84 %
– Point de fusion : 95 - 96°C
– Caractéristiques spectrales en IR :
  ν$CH_2$, $CH_3$ : 2980, 2920 $cm^{-1}$
  νCO ester : 1750 $cm^{-1}$
  νCO amide : 1645

**Example 5 : N - éthyl N - (4,6 - diméthylpyridin - 2 - yl) (6 -hydroxy - 2, 5, 7, 8 - tétraméthylchroman - 2 - yl) carboxamide**

Rendement : 86 %
– Point de fusion : 170 - 171°C
– Caractéristiques spectrales en IR :
  νOH : 3500, 3200 $cm^{-1}$
  ν$CH_2$, $CH_3$ : 2980, 2920, 2860 $cm^{-1}$
  νCO : 1620 $cm^{-1}$

**Example 6 : N - éthyl N - (4,6 dimethylpyridin - 2 - yl) (6 - méthoxy - 2, 5, 7, 8 - tétraméthylchroman - 2 - yl) carboxamide**

Rendement : 67,5 %
– Point de fusion : 90 - 92°C
– Caractéristiques spectrales en IR :
  ν$CH_3$ : 2970, 2920 $cm^{-1}$
  νCO : 1640 $cm^{-1}$

**Exemples 7 à 9 :**

En remplaçant dans l'exemple 1, stade B, la 2 - amino - 4,6 diméthylpyridine par la N - (4,6 - diméthylpyridin - 2 - yl) méthylamine, puis en opérant comme dans les exemples 2 et 3, on obtient successivement :

**Exemple 7 : N - méthyl N - (4,6 - diméthylpyridin - 2 - yl) (6 - acétoxy - 2, 5, 7, 8 - tétraméthylchroman - 2 - yl) carboxamide**

Rendement : 74 %
- Point de fusion : 133 - 134°C

**Exemple 8 : N - méthyl N - (4,6 diméthylpyridin - 2 - yl) (6 - hydroxy - 2, 5, 7, 8 - tétraméthylchroman - 2 - yl) carboxamide**

Rendement : 87 %
- Point de fusion : 141 - 142°C

**Exemple 9 : N - méthyl N - (4,6 diméthylpyridin - 2 - yl) (6 - méthoxy - 2, 5, 7, 8 - tétraméthylchroman - 2 - yl) carboxamide**

Rendement : 84 %
- Point de fusion : 135 - 136°C

**Exemples 10 à 12 :**

En remplaçant dans les exemples 3, 6, 9 l'iodure de méthyle par le bromo -1 propane on obtient respectivement, et de la même façon :

**Exemple 10 : N - (4,6 - diméthylpyridin - 2 - yl) (6 - propoxy - 2, 5, 7, 8 - tétraméthylchroman - 2 - yl) carboxamide**

Rendement : 77 %
- Caractéristiques spectrales en IR :
  - $\nu CH_2, CH_3$ : 2970, 2920, 2870 $cm^{-1}$
  - $\nu CO$ : 1670 $cm^{-1}$

**Exemple 11 : N - éthyl N - (4,6 - diméthylpyridin - 2 - yl) (6 - propoxy - 2, 5, 7, 8 - tétraméthylchroman - 2 - yl) carboxamide**

Rendement : 69 %
- Caractéristiques spectrales en IR :
  - $\nu CH_2, CH_3$ : 2970, 2910, 2880 $cm^{-1}$
  - $\nu CO$ : 1660 $cm^{-1}$

**Exemple 12 : N - méthyl N - (4,6 - diméthylpyridin - 2 - yl) (6 - propoxy - 2, 5, 7, 8 - tétraméthylchroman - 2 - yl) carboxamide**

Rendement : 72 %
- Caractéristiques spectrales en IR :
  - $\nu CH_2, CH_3$ : 2980, 2920, 2870 $cm^{-1}$
  - $\nu CO$ : 1670 $cm^{-1}$
  - $\nu C = C, C = N$ : 1620, 1560 $cm^{-1}$

**Exemples 13 à 15 :**

En remplaçant dans l'exemple 1 stade A l'anhydride acétique par le chlorure de l'acide triméthylacétique, puis en opérant comme dans les exemples 1, 4, et 7, au stade B, on obtient respectivement :

**Exemple 13 : N - (4,6 - diméthylpyridin - 2 - yl) (6 - triméthylacétoxy - 2, 5, 7, 8 - tétraméthylchroman - 2 - yl) carboxamide**

- Caractéristiques spectrales en IR :
  - $\nu NH$ : 3410 $cm^{-1}$
  - $\nu CH, CH_3$ : 2970, 2920, 2860 $cm^{-1}$
  - $\nu CO$ ester : 1750 $cm^{-1}$
  - $\nu CO$ amide = 1690 $cm^{-1}$

**Exemple 14 : N - éthyl N - (4,6 - diméthylpyridin - 2 - yl) (6 - triméthylacétoxy - 2, 5, 7, 8 - tétraméthylchroman - 2 - yl) carboxamide**

- Caractéristiques spectrales en IR :
  νCH, $CH_2$, $CH_3$ : 2970, 2910, 2850 $cm^{-1}$
  νCO ester = 1740 $cm^{-1}$
  νCO amide : 1695 $cm^{-1}$

**Exemple 15 : N - méthyl N - (4,6 diméthylpyridin - 2 - yl) (6 - triméthylacétoxy - 2, 5, 7, 8 - tétraméthylchroman - 2 - yl) carboxamide**

- Caractéristiques spectrales en IR :
  νCH, $CH_3$ : 2970, 2915, 2860 $cm^{-1}$
  νCO ester = 1750 $cm^{-1}$
  νCO amide = 1695 $cm^{-1}$

**Exemple 16 : N - (4,6 - diméthylpyridin - 2 - yl) (6 - hydroxy - 2, 5, 7, 8 - tétraméthylchroman - 2 - yl) méthylamine**

Dissoudre 2,2 grammes (6,2 mmole) du composé de l'exemple 2 dans 120 $cm^3$ de tétrahydrofuranne anhydre, ajouter 0,94 grammes d'hydrure de lithium et d'aluminium, chauffer à reflux pendant 3 heures.

Après refroidissement, verser le mélange sur de la glace, filtrer et extraire le produit par du chloroforme chaud. Sécher la phase organique sur sulfate de sodium. Après évaporation du solvant, triturer le résidu huileux obtenu avec de l'éther isopropylique. On obtient 1,7 grammes du produit de l'exemple :

– Point de fusion : 184 - 185°C
– Caractéristiques spectrales en IR :
  νNH : 3380 $cm^{-1}$
  ν$CH_2$, $CH_3$ : 2970, 2920 $cm^{-1}$

**Exemple 17 : N - (4,6 - diméthylpyridin - 2 - yl) (6 - méthoxy - 2, 5, 7, 8 - tétraméthylchroman - 2 - yl) méthylamine**

En remplaçant dans l'exemple 16, le composé de l'exemple 2 par le composé de l'exemple 3, on obtient de la même façon le composé de l'exemple 17 :
Rendement : 86 %
Point de fusion : 207 - 208°C

**Exemple 18 : N -( 4,6 - diméthylpyridin - 2 - yl) (6 - acétoxy - 2, 5, 7, 8 - tétraméthylchroman - 2 - yl) méthylamine, maléate**

En procédant comme dans l'exemple 1, stade A, mais en remplaçant l'acide (6-hydroxy 2,5,7,8-tetraméthylchroman-2-yl) carboxylique, par le composé de l'exemple 16, on obtient après salification par l'acide maléique, le composé de l'exemple 18 recristallisé de l'acétate d'éthyle.
Rendement : 62 %
Point de fusion : 157 - 158°C

**Exemples 19 à 21 :**

En remplaçant dans l'exemple 16 le composé de l'exemple 2 par le composé de l'exemple 5, et en procédant comme dans les exemples 16, 17 et 18 on obtient de la même façon respectivement :

**Exemple 19 : N - éthyl - N - (4,6 - diméthylpyridin - 2 - yl) (6 - hydroxy - 2, 5, 7, 8 - tétraméthylchroman - 2 - yl) méthylamine**

**Exemple 20 : N - éthyl - N - (4,6 - diméthylpyridin - 2 - yl) (6 - méthoxy - 2, 5, 7, 8 - tétraméthylchroman - 2 - yl) méthylamine**

**Exemple 21 : N - éthyl - N - (4,6 - diméthylpyridin - 2 - yl) (6 - acétoxy - 2, 5, 7, 8 - tétraméthylchroman - 2 - yl) méthylamine**

## ETUDE PHARMACOLOGIQUE DES COMPOSES DE L'INVENTION

**Les composés sont comparés avec le composé le plus proche de l'art antérieur qui est l'exemple 102 de la demande WO 88/08424 de formule (a) :**

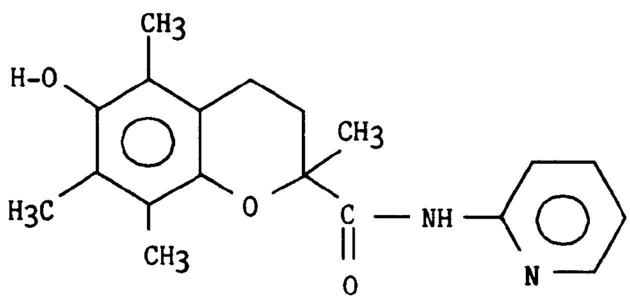

(a)

### Exemple 22 : ETUDE DE L'ACTIVITE ANTIPEROXYDANTE

L'action des composés de l'invention, susceptibles de piéger les radicaux ·OH a été étudiée d'une part, sur la peroxydation spontanée des lipides et d'autre part, sur la peroxydation induite par le système $Fe^{2+}$ - ascorbate (10 µM - 250 µM), et ce sur des homogénats de cerveaux de rats.

Lors de la mesure de la peroxydation lipidique spontanée, les homogénats de cerveau de rats sont placés en présence ou en absence des composés à tester durant 60 minutes à 37°C. La réaction est arrêtée à 0°C et le dosage du malondialdéhyde est effectué à l'aide d'acide thiobarbiturique par la méthode de YAGI, K (1976) Biochem. Med, 15, 212 - 216. La peroxydation lipidique est déterminée par les substances réagissant avec l'acide thiobarbiturique exprimées en nanomoles de malondialdéhyde.

Lors de la mesure de la peroxydation lipidique induite, la méthodologie est identique à la précédente à l'exception de l'addition à l'homogénat du système inducteur de radicaux, $Fe^{2+}$ - ascorbate. Les substances de référence sont le probucol et la vitamine E.

Les concentrations des composés testés inhibant de 50 % la peroxydation du substrat sont calculées.

Il est apparu que les composés de l'invention possèdent une activité antiperoxydante particulièrement intense puisque supérieure d'un facteur 100 à celle du composé le plus proche de l'art antérieur. Ce résultat très intéressant se produit que la peroxydation soit spontanée ou induite par un système chimique.

### Exemple 23 : ETUDE DU POUVOIR PROTECTEUR DE L'OXYDATION DES LDL

La capacité des composés de l'invention à diminuer les proportions de LDL oxydées a été mesurée de la façon suivante. On réalise une incubation de 24 heures regroupant des LDL natives, un système $Cu^{2+}$ générateur de radicaux libres et les composés à tester.

Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol.

Il apparaît clairement que les composés de l'invention ont un pouvoir protecteur très important et significativement supérieur à celui du composé le plus proche de l'art antérieur.

A titre de comparaison, pour une concentration de $10^{-5}$ **M**, le niveau de protection obtenu pour les composés de l'invention dépasse celui du probucol et est plus de 5 fois supérieur à celui du composé le plus proche de l'art antérieur.

**Exemple 24 : ETUDE DE L'ACTIVITE INHIBITRICE DE LA BIOSYNTHESE DES PROSTANOIDES**

La recherche de l'activité inhibitrice des composés sur la biosynthèse des prostanoïdes a été réalisée sur des plaquettes humaines préalablement activées par la thrombine et mises en présence des produits à tester.

La production de thromboxane $B_2$, un des prostanoïdes majeurs synthétisés par les plaquettes, est déterminée par dosage radioimmunologique (RIA).

Les composés de l'invention inhibent la production du thromboxane $B_2$ de façon très importante alors que le composé le plus proche de l'art antérieur n'a aucun effet sur cette production.

A titre d'exemple, pour une concentration de $10^{-5}$**M**, les composés de l'invention provoquent une inhibition de la production du thromboxane $B_2$ de l'ordre de 80 %.

**Exemple 25 : COMPOSITION PHARMACEUTIQUE : COMPRIMES**

Comprimés dosés à 50 mg de N-(4,6 - diméthylpyridin - 2 - yl) (6 - hydroxy 2, 5, 7, 8 - tétraméthylchroman - 2 - yl) carboxamide.

Formule de préparation pour 1 000 comprimés :

| | |
|---|---|
| N - (4,6 - diméthylpyridin - 2 - yl) (6 - hydroxy 2, 5, 7, 8 - tétraméthylchroman 2 - yl) carboxamide | 50g |
| Amidon de blé | 15g |
| Amidon de maïs | 15g |
| Lactose | 65g |
| Stéarate de Magnesium | 2g |
| Silice | 1g |
| Hydroxypropylcellulose | 2g |

**Revendications**

**1)** Dérivés de formule générale I

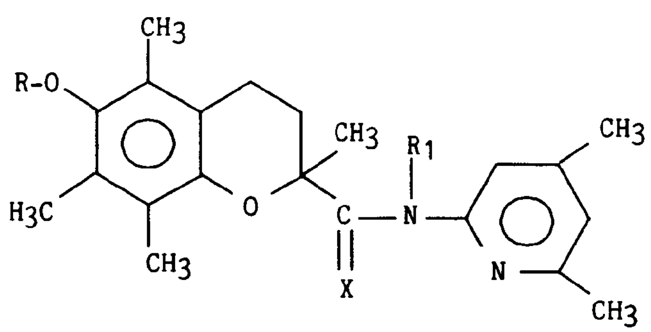

I

dans laquelle :

$R_1$ représente un atome d'hydrogène ou un radical alkyle comprenant de 1 à 3 atomes de carbone,

R représente un atome d'hydrogène, un radical alkyle comprenant de 1 à 4 atomes de carbone, ou un radical acyle - CO - R' dans lequel R' représente un radical alkyle comportant de 1 à 4 atomes de carbone,

X représente O ou $H_2$,

leurs isomères,

ainsi que, lorsque X représente $H_2$, leurs sels d'addition à un acide pharmaceutiquement acceptable, et lorsque R représente un atome d'hydrogène, leurs sels d'addition à une base pharmaceutiquement acceptable.

**2)** Dérivés selon la revendication 1 pour lesquels R représente un atome d'hydrogène, leurs isomères et

leurs sels d'addition à une base où à un acide pharmaceutiquement acceptables.

**3)** Dérivés selon la revendication 2 pour lesquels X représente un atome d'oxygène, leurs isomères, et leurs sels d'addition à une base pharmaceutiquement acceptable.

**4)** Le composé selon la revendication 1 qui est le N - (4,6 - diméthylpyridin - 2 - yl) (6 - hydroxy - 2, 5, 7, 8 - tétraméthylchroman - 2 - yl) carboxamide, ses isomères, et ses sels d'addition à une base pharmaceutiquement acceptable.

**5)** Le composé selon la revendication 1 qui est le N - éthyl - N - (4,6 - diméthylpyridin - 2 - yl) (6 - hydroxy - 2, 5, 7, 8 - tétraméthylchroman - 2 - yl) carboxamide, ses isomères, et ses sels d'addition à une base pharmaceutiquement acceptable.

**6)** Le composé selon la revendication 1 qui est le N - méthyl - N - (4, 6 - diméthylpyridin - 2 - yl) (6 - hydroxy - 2, 5, 7, 8 - tétraméthylchroman - 2 - yl) carboxamide, ses isomères, et sels d'addition à une base pharmaceutiquement acceptable.

**7)** Le composé selon la revendication 1, qui est le N-(4,6-diméthylpyridin-2-yl) (6-hydroxy-2,5,7,8-tétraméthylchroman-2-yl)méthylamine, ses isomères, et ses sels d'addition à une base ou à un acide pharmaceutiquement acceptable.

**8)** Procédé d'obtention des dérivés de formule I selon la revendication 1, caractérisé en ce que l'on estérifie l'acide (6 - hydroxy - 2, 5, 7, 8 - tétraméthylchroman - 2 - yl)carboxylique, en milieu basique anhydre par un anhydride d'acide de formule R' - CO - O - CO - R', ou un halogénure d'acide de formule R' - CO - Hal, formules dans lesquelles R' a la même signification que dans la formule I et Hal représente un atome d'halogène, pour obtenir un acide de formule II

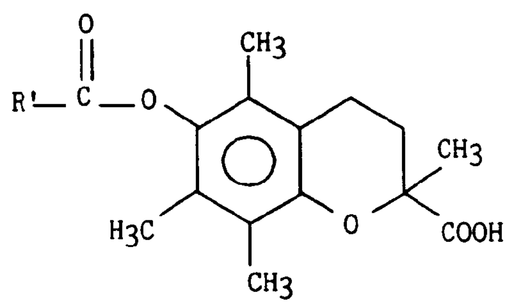

II

dans laquelle R' a la même définition que dans la formule I, qui est transformé en son chlorure par action du chlorure de thionyle en milieu anhydre, puis traité, dans un solvant approprié en présence d'un agent alcalin, par une amine de formule III

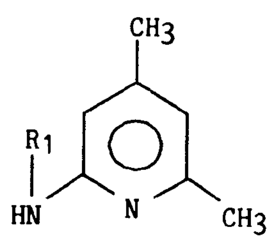

III

dans laquelle $R_1$ a la même signification que dans la formule I, pour obtenir un composé de formule $I_a$

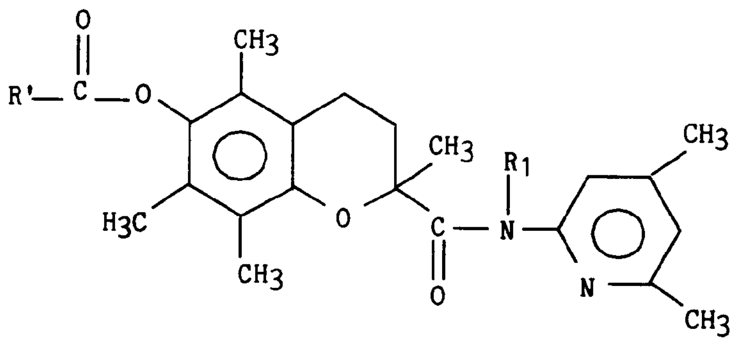

Ia

dans laquelle R′ et $R_1$ sont tels que définis précédemment, cas particulier des composés de formule I pour lesquels R représente - CO - R′ et X représente un atome d'oxygène, qui est ensuite saponifié par action d'un hydroxyde d'un métal alcalin ou alcalino-terreux en un composé de formule $I_b$

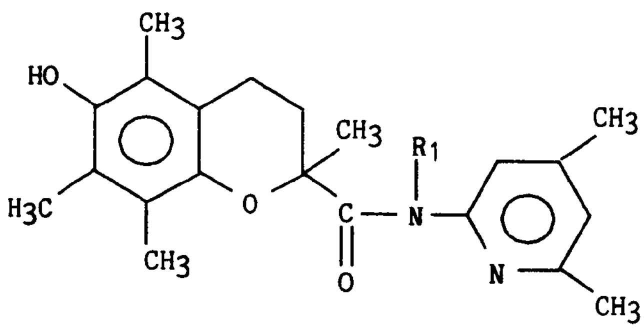

Ib

dans laquelle $R_1$ est tel que défini précédemment, cas particulier des composés de formule I pour lesquels R représente un atome d'hydrogène et X un atome d'oxygène qui peut être ensuite :

– soit transformé en son sel d'addition à une base pharmaceutiquement acceptable,

– soit éthérifié par un dérivé halogéné de formule $R_2$ - Hal, dans laquelle $R_2$ représente un radical alkyle de 1 à 4 atomes de carbone et Hal représente un atome d'halogène pour obtenir un composé de formule $I_c$

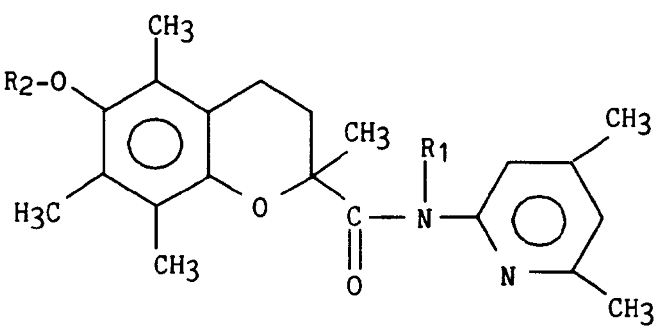

Ic

dans laquelle $R_1$ et $R_2$ sont tels que définis précédemment, cas particulier des composés de formule I pour lesquels X représente un atome d'oxygène et R représente un groupement $R_2$ tel que défini précédemment,

– soit réduit par action d'un hydrure mixte de métal alcalin en un dérivé de formule $I_d$

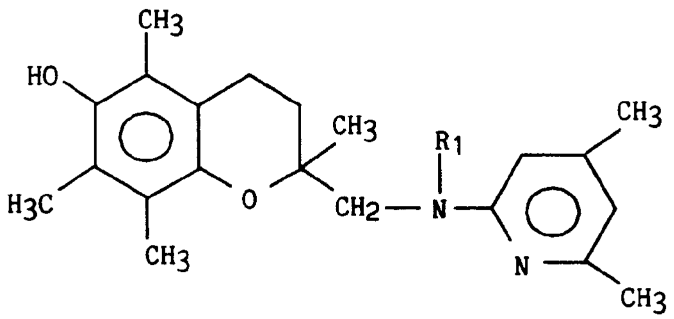

Id

dans laquelle $R_1$ est telle que défini précédemment, cas particulier des composés de formule I pour lesquels R représente un atome d'hydrogène et X représente $H_2$,
que l'on peut,

– ou bien salifier par action d'une base ou d'un acide pharmaceutiquement acceptable,

– ou bien éthérifier ou estérifier par action d'un dérivé de formule $R'_2$ - Hal dans laquelle Hal représente un atome d'halogène et $R'_2$ représente un radical alkyle de 1 à 4 atomes de carbone ou un radical acyle - CO - R' tel que défini dans la formule I, en un dérivé de formule $I_e$

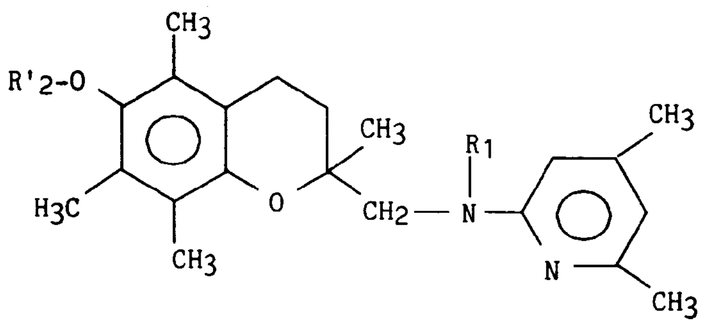

Ie

dans laquelle $R_1$ et $R'_2$ sont tels que définis précédemment, cas particulier des composés de formule I pour lesquels X représente $H_2$ et R possède les significations de $R'_2$, qui est, si l'on désire, salifié par action d'un acide pharmaceutiquement acceptable,
les composés de formule $I_a$, $I_b$, $I_c$, $I_d$, et $I_e$ formant l'ensemble des composés de formule I, qui peuvent être, si l'on désire, séparés en leurs isomères selon des techniques classiques de séparation.

**9)** Composition pharmaceutique contenant comme principe actif un composé selon l'une des revendications 1 à 7, seul ou en combinaison avec un ou plusieurs excipients inertes non toxiques.

**10)** Composition pharmaceutique selon la revendication 9 utilisable dans le traitement et la prévention des affections dues ou reliées à des phénomènes de peroxydation et de dérèglement de la synthèse des prostanoïdes, c'est à dire les désordres ischémiques centraux ou périphériques, les maladies inflammatoires, les maladies métaboliques : l'athérome et l'artériosclérose, les maladies respiratoires : l'asthme ou l'emphysème, la maladie d'origine immunologique : le lupus érythémateux, les réactions allergiques, certains cancers, le vieillissement cérébral ou cutané, ainsi que dans la prévention et le traitement des dommages dus aux traumatismes chirurgicaux tels que la reperfusion d'organes.

**Revendications pour les Etats contractants suivants : ES, GR**

**1)** Procédé de préparation des dérivés de formule générale I

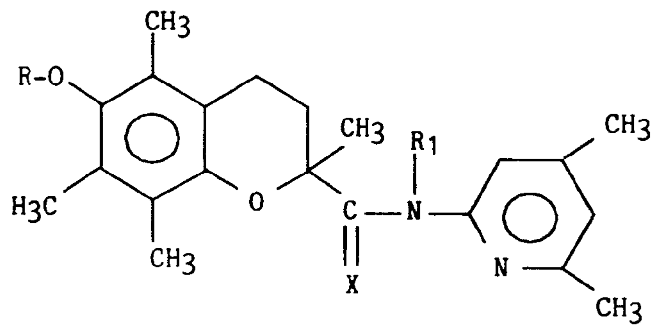

I

dans laquelle :

$R_1$ représente un atome d'hydrogène ou un radical alkyle comprenant de 1 à 3 atomes de carbone,

R représente un atome d'hydrogène, un radical alkyle comprenant de 1 à 4 atomes de carbone, ou un radical acyle - CO - R' dans lequel R' représente un radical alkyle comportant de 1 à 4 atomes de carbone,

X représente O ou $H_2$,

de leurs isomères,

ainsi que, lorsque X représente $H_2$, de leurs sels d'addition à un acide pharmaceutiquement acceptable, et lorsque R représente un atome d'hydrogène, de leurs sels d'addition à une base pharmaceutiquement acceptable, caractérisé en ce que :

on estérifie l'acide (6 - hydroxy - 2, 5, 7, 8-tétraméthylchroman - 2 - yl) carboxylique, en milieu basique anhydre par un anhydride d'acide de formule R' - CO - O - CO - R', ou un halogénure d'acide de formule R' - CO - Hal, formules dans lesquelles R' a la même signification que dans la formule I et Hal représente un atome d'halogène, pour obtenir un acide de formule II

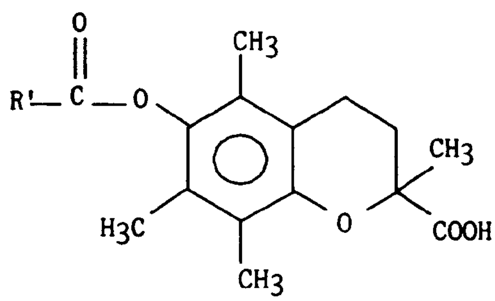

II

dans laquelle R' a la même définition que dans la formule I, qui est transformé en son chlorure par action du chlorure de thionyle en milieu anhydre, puis traité, dans un solvant approprié en présence d'un agent alcalin, par une amine de formule III

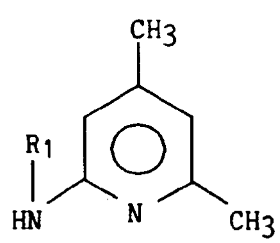

III

dans laquelle $R_1$ a la même signification que dans la formule I, pour obtenir un composé de formule $I_a$

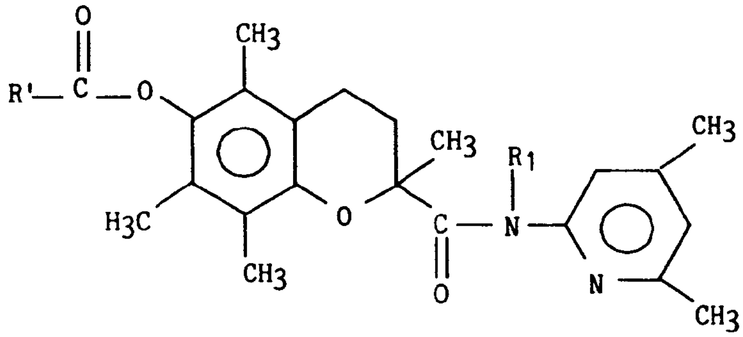

$I_a$

dans laquelle R′ et $R_1$ sont tels que définis précédemment, cas particulier des composés de formule I pour lesquels R représente - CO - R′ et X représente un atome d'oxygène, qui est ensuite saponifié par action d'un hydroxyde d'un métal alcalin ou alcalino-terreux en un composé de formule $I_b$

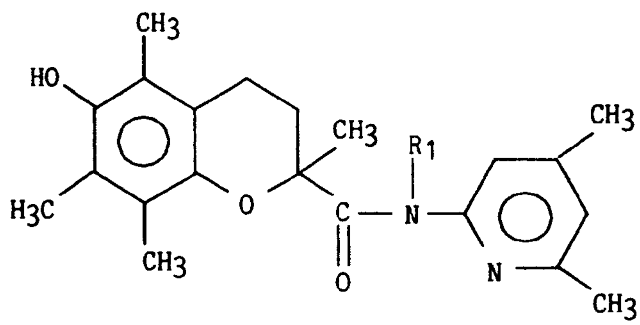

$I_b$

dans laquelle $R_1$ est tel que défini précédemment, cas particulier des composés de formule I pour lesquels R représente un atome d'hydrogène et X un atome d'oxygène qui peut être ensuite :

– soit transformé en son sel d'addition à une base pharmaceutiquement acceptable,

– soit éthérifié par un dérivé halogéné de formule $R_2$ - Hal, dans laquelle $R_2$ représente un radical alkyle de 1 à 4 atomes de carbone et Hal représente un atome d'halogène pour obtenir un composé de formule $I_c$

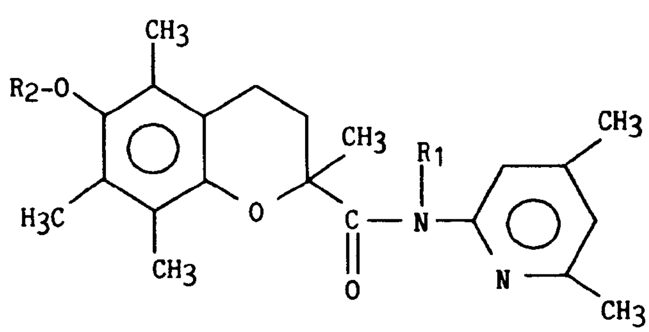

$I_c$

dans laquelle $R_1$ et $R_2$ sont tels que définis précédemment, cas particulier des composés de formule I pour lesquels X représente un atome d'oxygène et R représente un groupement $R_2$ tel que défini précédemment,

– soit réduit par action d'un hydrure mixte de métal alcalin en un dérivé de formule $I_d$

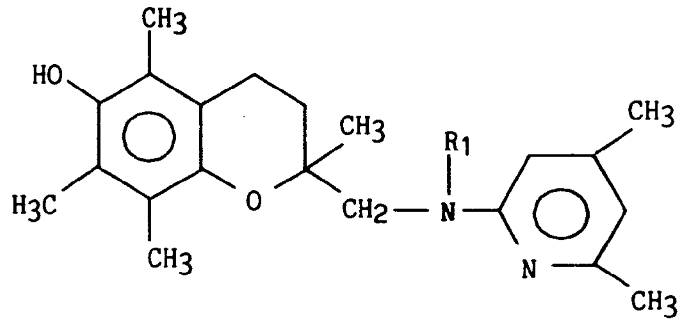

Id

dans laquelle $R_1$ est telle que défini précédemment, cas particulier des composés de formule I pour lesquels R représente un atome d'hydrogène et X représente $H_2$, que l'on peut,

– ou bien salifier par action d'une base ou d'un acide pharmaceutiquement acceptable,

– ou bien éthérifier ou estérifier par action d'un dérivé de formule $R'_2$ - Hal dans laquelle Hal représente un atome d'halogène et $R'_2$ représente un radical alkyle de 1 à 4 atomes de carbone ou un radical acyle - CO - R' tel que défini dans la formule I, en un dérivé de formule $I_e$

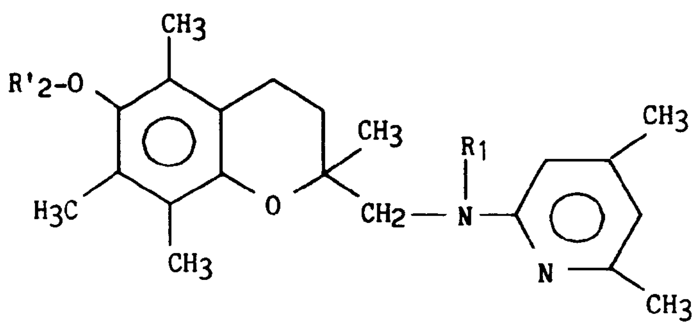

Ie

dans laquelle $R_1$ et $R'_2$ sont tels que définis précédemment, cas particulier des composés de formule I pour lesquels X représente $H_2$ et R possède les significations de $R'_2$, qui est, si l'on désire, salifié par action d'un acide pharmaceutiquement acceptable,

les composés de formule $I_a$, $I_b$, $I_c$, $I_d$ et $I_e$ formant l'ensemble des composés de formule I, qui peuvent être, si l'on désire, séparés en leurs isomères selon des techniques classiques de séparation.

**2)** Procédé de préparation selon la revendication 1 des dérivés de formule (I) pour lesquels R représente un atome d'hydrogène, de leurs isomères, et de leurs sels d'addition à une base ou à un acide pharmaceutiquement acceptables.

**3)** Procédé de préparation selon la revendication 1 des dérivés de formule I pour lesquels R représente un atome d'hydrogène et X représente un atome d'oxygène, de leurs isomères, et de leurs sels d'addition à une base pharmaceutiquement acceptable.

**4)** Procédé de préparation selon la revendication 1 du composé qui est le N- (4,6 - diméthylpyridin - 2 - yl) (6 - hydroxy - 2, 5, 7, 8 - tétraméthylchroman - 2 - yl) carboxamide, de ses isomères, et de ses sels d'addition à une base pharmaceutiquement acceptable.

**5)** Procédé de préparation selon la revendication 1 du composé 1 qui est le N - éthyl - N - (4,6 - diméthylpyridin - 2 - yl) (6 - hydroxy - 2, 5, 7, 8 - tétraméthylchroman - 2 - yl) carboxamide, de ses isomères, et de ses sels d'addition à une base pharmaceutiquement acceptable.

**6)** Procédé de préparation selon la revendication 1 du composé qui est le N- méthyl - N - (4,6 - diméthylpyridin - 2 - yl) (6 - hydroxy - 2, 5, 7, 8 - tétraméthylchroman - 2 - yl) carboxamide, de ses isomères, et de ses sels d'addition à une base pharmaceutiquement acceptable.

**7)** Procédé de préparation selon la revendication 1 du composé qui est le N-(4,6-diméthylpyridin-2-yl) (6-hydroxy-2,5,7,8-tétraméthylchroman-2-yl)méthylamine, de ses isomères, et de ses sels d'addition à une base ou à un acide pharmaceutiquement acceptable.

**8)** Procédé de préparation d'une composition pharmaceutique contenant comme principe actif un composé préparé selon l'une des revendications 1 à 7, seul ou en combinaison avec un ou plusieurs excipients inertes

non toxiques.

**9)** Procédé de préparation d'une composition pharmaceutique selon la revendication 8 utilisable dans le traitement et la prévention des affections dues ou reliées à des phénomènes de peroxydation et de dérèglement de la synthèse des prostanoïdes, c'est à dire les désordres ischémiques centraux ou périphériques, les maladies inflammatoires, les maladies métaboliques : l'athérome et l'artériosclérose, les maladies respiratoires : l'asthme ou l'emphysème, la maladie d'origine immunologique : le lupus érythémateux, les réactions allergiques, certains cancers, le vieillissement cérébral ou cutané, ainsi que dans la prévention et le traitement des dommages dus aux traumatismes chirurgicaux tels que la reperfusion d'organes.

Office européen des brevets

# RAPPORT DE RECHERCHE EUROPEENNE

Numero de la demande

EP 92 40 0577

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 412 939 (CIBA-GEIGY AG)<br>* page 3 - page 4, ligne 20 * | 1,9 | C07D405/12<br>A61K31/35 |
| A | EP-A-0 404 197 (KYOWA HAKKO KOGYO CO.,LTD)<br>* page 3 - page 4, ligne 30 * | 1,9 | |
| A | EP-A-0 342 664 (G.D.SEARLE & CO.)<br>* page 3 - page 6, ligne 7 * | 1,9 | |
| A | EP-A-0 335 375 (WARNER-LAMBERT COMPANY)<br>* page 2 - page 4, ligne 11 * | 1,9 | |
| A | EP-A-0 180 190 (KURARAY CO.,LTD)<br>* page 1 - page 7, alinéa 3 * | 1,9 | |
| A | PATENT ABSTRACTS OF JAPAN<br>vol. 11, no. 297 (C-448)(2744) 25 Septembre 1987<br>& JP-A-62 089 676 ( KURARAY CO.,LTD ) 24 Avril 1987<br>* abrégé * | 1,9 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| BERLIN | 14 MAI 1992 | KYRIAKAKOU G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)